# EUROPEAN PATENT APPLICATION

(11) **EP 2 848 245 A1**
(43) Date of publication of application: **18.03.2015**
(21) Application number: 13788233.8
(22) Date of filing: 10.05.2013
(51) Int. Cl.: A61K 9/22, A61K 9/20, A61K 31/506

(54) **BOSENTAN CONTROLLED RELEASE ORAL PREPARATION**

(30) Priority: 11.05.2012 KR 20120050125
(71) Applicant: Hanall Biopharma Co., Ltd., Daejeon 306-120 (KR)
(72) Inventor: KIM, Sung Wuk, Seongnam-si Gyeonggi-do 463-802 (KR); CHOI, Seong Choon, Seoul 121-020 (KR); KOO, Ja Seong, Daejeon 305-756 (KR); SUN, Sang Ouk, Daejeon 305-509 (KR); LEE, Na Young, Daejeon 305-509 (KR)
(74) Representative: Vossius & Partner
(86) International application number: PCT/KR2013/004182
(87) International publication number: WO 2013/169082

(57) **Abstract**

Provided are an extended release preparation for oral administration of bosentan including bosentan, a pharmaceutically acceptable salt thereof or a solvate thereof as an active ingredient and a hydrophilic polymer as a sustained release excipient, and a method of manufacturing the same. Accordingly, the number of administrations of the bosentan, a pharmaceutically acceptable salt thereof or a solvate thereof may be reduced, thereby enhancing ease of administration and patient compliance. In addition, the extended release preparation for oral administration of bosentan may be effectively manufactured.

## Description

### [Technical Field]

The present invention relates to an extended release preparation for oral administration of bosentan, and more particularly, to a new extended release preparation for oral administration of bosentan capable of enhancing ease of administration.

### [Background Art]

Bosentan, the first endothelin receptor antagonist (ERA) drug preventing reduction and worsening of exercise ability triggered by pulmonary hypertension, is a compound having the chemical name of 4-tert-butyl-N-[6-(2-hydroxy-ethoxy)-5-(2-methoxy-phenoxy)-2-(pyrimidin-2-yl)-pyrimidin-4-yl]-benzenesulfonamide represented by Formula 1, and is a representative pulmonary hypertension agent disclosed in European Patent No. 0526708 A1 which is now commercially available under the product name of Tracleer tablets (Actelion). Bosentan is mainly a drug for patients in pulmonary hypertension functional classifications II, III and IV, and is administered twice a day (morning and evening).

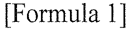

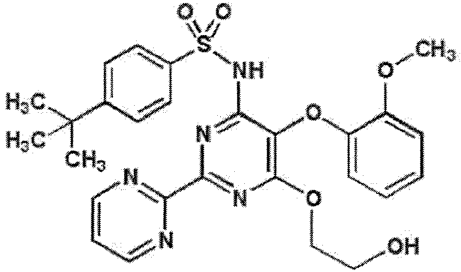

Bosentan is a poorly soluble drug that is not dissolved in water, and thus 100 ml or more of water is needed to dissolve 0.1 mg of bosentan at pH 1.0, and 50 ml or more of water is needed to dissolve 0.1 mg of bosentan at pH 5.0. However, bosentan has a physiochemical characteristic such that approximately 0.3 ml of water is needed to dissolve 0.1 mg of bosentan at pH 7.5. In other words, as pH increases, the solubility of the drug increases.

In addition, bosentan has a short half-life and thus is administered twice a day.

Such twice-a-day administration is inconvenient, resulting in reduction in patient compliance, missing of suitable administration times, and further increasing possibility of the occurrence of side effects, and therefore solutions to these problems need to be found. However, there have been many difficulties in developing a therapeutic preparation that addresses the problems due to the low solubility of a bosentan-containing drug.

Meanwhile, in Korean Patent Publication No. 2008-0014002, technology relating to preparation of a dispersible tablet of bosentan that can be provided to pediatric patients with pulmonary hypertension is disclosed. This technology is about a preparation that is convenient for pediatric patients and improves ease of administration or solubility. As described above, bosentan is a poorly soluble drug, and is generally studied with regard to the development of formulating technology to improve ease of administration through improved dispersibility or to improve absorption through improved solubility.

### [Disclosure]

### [Technical Problem]

The present invention is directed to providing a new extended release preparation for oral administration of bosentan capable of enhancing ease of administration.

The present invention is also directed to providing a method of manufacturing an extended release preparation for oral administration of bosentan capable of enhancing ease of administration.

The present invention is also directed to providing a method of treating a patient with pulmonary hypertension using a new extended release preparation for oral administration of bosentan.

Hereinafter, other objects that are not described above will be clearly understood by those of ordinary skill in the art according to the following descriptions.

### [Technical Solution]

The inventors applied a sustained release excipient to bosentan that was solely developed to improve ease of administration through improved dispersibility or absorption through improved solubility, and thereby a new preparation for oral administration capable of improving ease of administration was completed.

According to an exemplary embodiment of the present invention, an extended release preparation for oral administration of bosentan includes bosentan, or a pharmaceutically acceptable salt or solvate thereof as an active ingredient, and a sustained release excipient.

The active ingredient may be bosentan monohydrate.

The sustained release excipient may be at least one selected from a hydrophilic polymer, a water-insoluble polymer, and a lipid material.

The hydrophilic polymer may be at least one selected from hydroxyethyl cellulose, hydroxypropyl cellulose, hydroxypropyl methylcellulose, polyvinylpyrrolidone, polyvinyl alcohol, polyethylene glycol, polyethylene oxide and a carbomer.

The water-insoluble polymer may be at least one selected from ethylcellulose, cellulose acetate, a poly(ethylacrylate, methyl methacrylate) copolymer, and a poly(ethylacrylate, methyl methacrylate, trimethylammonioethyl methacrylate) copolymer.

The lipid material may be at least one selected from glyceryl behenate, glyceryl palmitostearate, glyceryl oleate, glyceryl stearate and cetylalcohol.

The sustained release excipient may be included at 0.5 to 200 parts by weight with respect to 100 parts by weight of the active ingredient.

The extended release preparation for oral administration of bosentan may have a dissolution rate of the active ingredient after one hour of 10 to 50%, a dissolution rate of the active ingredient after 2 hours of 20 to 70%, and a dissolution rate of the active ingredient after 12 hours of 60% or more in a dissolution test by a paddle method using a phosphate buffer (pH 7.7) as a dissolution medium at a stirring speed of 50 rpm.

The extended release preparation for oral administration of bosentan may have a dissolution rate of the active ingredient after one hour of 10 to 50%, a dissolution rate of the active ingredient after 10 hours of 50% or more, and a dissolution rate of the active ingredient after 24 hours of 80% or more in a dissolution test by a paddle method using distilled water containing 1.0% sodium lauryl sulfate as a dissolution medium at a stirring speed of 50 rpm.

The extended release preparation for oral administration of bosentan may be a tablet.

The tablet may be a plain tablet or a coated tablet.

The extended release preparation for oral administration of bosentan may be suitable for administration once-a-day.

The extended release preparation for oral administration of bosentan may include an extended-release section containing the active ingredient and the sustained release excipient; and a non-extended-release section containing the same active ingredient as that contained in the extended-release section and discriminated from the extended-release section.

The active ingredient contained in the non-extended-release section is contained at 0.1 to 50 parts by weight, and the active ingredient contained in the extended-release section is contained at 50 to 99.9 parts by weight with respect to 100 parts by weight of the total active ingredients in the bosentan extended-release oral preparation.

The non-extended-release section may be immediately released.

The non-extended-release section may further include a disintegrant.

An enteric coating layer containing an enteric polymer may be further formed on a surface of the extended-release section.

The enteric polymer may be at least one selected from hydroxypropyl methylcellulose acetate succinate, hydroxypropyl methylcellulose phthalate, cellulose acetate phthalate, a 1:1 copolymer of poly(methacrylic acid, methylmethacrylate), cellulose acetate phthalate, a 1:2 copolymer of poly(methacrylic acid, methylmethacrylate) and a 1:1 copolymer of poly(methacrylic acid, ethylmethacrylate).

The enteric polymer may be contained at 1 to 40 parts by weight with respect to 100 parts by weight of the extended-release section.

The extended release preparation for oral administration of bosentan may be a tablet.

The tablet may be a press-coated tablet in which the extended-release section may become a core and the non-extended-release section may become a shell.

The tablet may be a multiple layer tablet in which the extended-release section and the non-extended-release section may be formed in respective layers.

The multiple layer tablet may include a placebo layer not containing an active ingredient, and the placebo layer may have a separate layer in addition to the respective layers formed by the extended release section and the non-extended-release section.

The tablet may be a monolayer tablet.

In addition, a method of manufacturing an extended release preparation for oral administration of bosentan according to an exemplary embodiment of the present invention may include preparing bosentan, a pharmaceutically acceptable salt thereof or a solvent thereof as an active ingredient; manufacturing an extended-release section containing a sustained release excipient and a part of the active ingredient prepared in the first step; manufacturing a non-extended-release section containing the remaining content excluding the active ingredient contained in the extended-release section formed in the second step of the active ingredient prepared in the first step as an active ingredient; and formulating the extended-release section formed in the second step and the non-extended-release section formed in the third step into a single formulation.

Alternatively, the method of manufacturing an extended release preparation for oral administration of bosentan according to an exemplary embodiment of the present invention may include (A) preparing bosentan, a pharmaceutically acceptable salt thereof, or a solvate thereof as an active ingredient; (B) manufacturing a extended-release granule containing a sustained release excipient and a part of the active ingredient prepared in step (A); (C) manufacturing a extended-release core by compressing the granule manufactured in step (B); (D) manufacturing a non-extended-release granule containing the remaining content excluding the active ingredient contained in the granule manufactured in step (B) of the active ingredient prepared in step (A); and (E) obtaining a press-coated tablet by compressing the extended-release core manufactured in step (C) that is encompassed by the non-extended- release granule prepared in step (D).

A content of the active ingredient in step (B) may be 50 to 99.9 parts by weight, and a content of the active ingredient in step (D) may be 0.1 to 50 parts by weight with respect to 100 parts by weight of the active ingredient prepared in step (A).

The extended-release core manufactured in step (C) may further undergo enteric coating after the compression of the granule manufactured in step (B).

In addition, in another example, a method of manufacturing an extended release preparation for oral administration of bosentan according to one exemplary embodiment of the present invention may include (a) preparing bosentan, a pharmaceutically acceptable salt thereof, or a solvate thereof as an active ingredient; (b) manufacturing an extended-release granule containing a sustained release excipient and a part of the active ingredients prepared in step (a); (c) manufacturing a non-extended-release granule containing the remaining content excluding the active ingredient contained in the granule manufactured in step (b) of the active ingredient prepared in step (a) as an active ingredient; and (d) obtaining a multiple layer tablet by compressing the extended-release granule manufactured in step (b) and the non-extended-release granule manufactured in step (c) in layers.

The multiple layer tablet may include a placebo layer, and may be prepared by adding a granule for a placebo layer and compressing the granule for a placebo layer together with the extended-release granule and the non-extended-release granule in step (d). At this time, the granule for a placebo layer may be compressed and formed in a separate layer from the layers of the extended-release granule and the non-extended-release granule.

In addition, in still another aspect, a method of manufacturing an extended release preparation for oral administration of bosentan according to one exemplary embodiment of the present invention may include preparing bosentan, a pharmaceutically acceptable salt thereof, or a solvate thereof as an active ingredient; preparing a sustained release excipient; and mixing the active ingredient prepared in the first step with the sustained release excipient prepared in the second step to be formulated into a single formulation.

In addition, a method of treating pulmonary hypertension according to an exemplary embodiment of the present invention may include administering an extended release preparation for oral administration of bosentan according to an exemplary embodiment of the present invention to mammals including humans in need of administration once a day.

### [Advantageous Effects]

According to the present invention, the number of administrations of bosentan, a pharmaceutically acceptable salt thereof or a solvate thereof can be reduced, thereby enhancing ease of administration and patient compliance. In addition, the therapeutic effect can be increased and the side effect can be reduced. Moreover, the extended release preparation for oral administration of bosentan according to the present invention can be effectively prepared.

### [Description of Drawings]

FIG. 1 is a graph showing results of a dissolution test for the extended release preparation for oral administration of bosentan according to Examples 1 to 3.
FIG. 2 is a graph showing results of a dissolution test for the extended release preparation for oral administration of bosentan according to Examples 4 and 5.
FIG. 3 is a graph showing results of a dissolution test for the extended release preparation for oral administration of bosentan according to Examples 6 and 7.
FIG. 4 is a graph showing results of a dissolution test for the extended release preparation for oral administration of bosentan according to Examples 11 and 12.
FIG. 5 is a graph showing results of a dissolution test for the extended release preparation for oral administration of bosentan according to Examples 13 and 14.
FIG. 6 is a graph showing results of a dissolution test for the extended release preparation for oral administration of bosentan according to Examples 18 and 20.
FIG. 7 is a graph showing results of a dissolution test for the extended release preparation for oral administration of bosentan according to Examples 22 and 23.
FIG. 8 is a flowchart illustrating a method of manufacturing the extended release preparation for oral administration of bosentan according to an exemplary embodiment of the present invention.

### [Modes of the Invention]

The advantages and characteristics of the present invention and methods of achieving the same will be clear with reference to the accompanying drawings and Examples that will be described below. However, the present invention is not limited to the Examples disclosed below, but may be embodied in various different forms, and the Examples are provided only to make the disclosure of the present invention complete and to completely convey the scope of the present invention to those of ordinary skill in the art, with the present invention being defined only by the claims. Unless separately cited throughout the specification, "bosentan" generally includes bosentan of Formula 1 {4-tert-butyl-N-[6-(2-hydroxy-ethoxy)-5-(2-methoxy-phenoxy)-2-(pyrimidin-2-yl)-pyrimidin-4-yl]-benzensulfonamide}, a pharmaceutically acceptable salt thereof, or a solvate thereof. That is, bosentan also includes salt types such as bosentan hydrogen sulfate, bosentan maleate, etc., and solvate types including hydrate types such as bosentan monohydrate. Bosentan monohydrate is generally used as bosentan, but the present invention is not limited thereto.

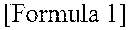

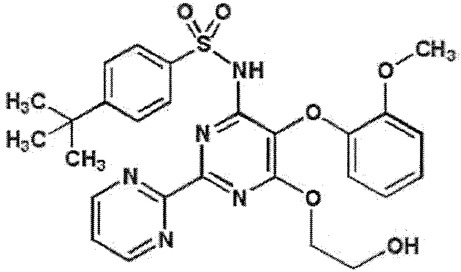

The "extended release preparation for oral administration" means a preparation in which emission of an active ingredient is controlled according to a desire among pharmaceutical preparations capable of being orally administered to mammals including humans.

The "extended-release" means releasability in such a type that an active ingredient is released slowly over a long time, for example, in a zero-order release type, to maintain a drug effect for a long time.

The "sustained release excipient" means a material added to a preparation containing an active ingredient to be extended-released, such as a hydrophilic polymer, a water-insoluble polymer or a lipid material depending on solubility towards water and characteristics.

The "hydrophilic polymer" means a pharmaceutical additive that dissolves in water or absorbs water to generate viscosity, and may be, but is not limited to, at least one selected from hydroxyethylcellulose, hydroxypropylcellulose, hydroxypropylmethylcellulose, polyvinylpyrrolidone, polyvinylalcohol, polyethylene glycol, polyethylene oxide, and a carbomer.

The "water-insoluble polymer" means a pharmaceutical additive that does not dissolve in water, and may be, but is not limited to, at least one selected from ethylcellulose, celluloseacetate, a poly(ethylacrylate, methylmethacrylate) copolymer, and a poly(ethylacrylate, methylmethacrylate, trimethylammonioethyl methacrylate) copolymer.

The "lipid material" may mean a material that does not mixed with water, and may be at least one selected from glyceryl behenate, glyceryl palmitostearate, glyceryl oleate, glyceryl stearate, and cetylalcohol.

The "non-extended-release" means a type in which an active ingredient is released over a shorter time than the extended-release and absorbed. The non-extended-release may be immediate release, and the immediate release means that a preparation is rapidly integrated, and thus an active ingredient is rapidly dissolved or absorbed. To be immediate release, a disintegrant may be further included in the preparation.

The "enteric polymer" is a pH-dependent polymer, which does not dissolve at a low pH, but dissolves at a higher pH, and may be, but is not limited to, at least one selected from hydroxypropyl methylcellulose acetate succinate (HPMC-AS), hydroxypropyl methylcellulose phthalate (HPMCP), cellulose acetate phthalate, a 1:1 copolymer of poly(methacrylic acid, methylmethacrylate), a 1:2 copolymer of poly(methacrylic acid, methylmethacrylate), and a 1:1 copolymer of poly(methacrylic acid, ethyl methacrylate).

Hereinafter, the present invention will be described in further detail.

An extended release preparation for oral administration of bosentan according to an exemplary embodiment of the present invention may include bosentan, a pharmaceutically acceptable salt thereof or a solvate thereof as an active ingredient, and preferably includes a sustained release excipient, and thus poorly soluble bosentan can be extended-released by the sustained release excipient. At this time, as at least one selected from a hydrophilic polymer, a water-insoluble polymer and a lipid material is applied as the sustained release excipient, going of the solubility of bosentan to a rate-determining step is avoided, a desired extended-release aspect can be obtained, and a releasing rate can be variously controlled, resulting in extended-release.

The sustained release excipient may be included in a content of 0.5 to 200 parts by weight, and preferably 1 to 150 parts by weight with respect to 100 parts by weight of the active ingredient. It may be difficult to obtain a sustained release pattern below the above range, and the active ingredient may be lost by excretion rather than absorption due to excessive delay of release above the above range.

Through the above-described composition, extended- release of the bosentan is possible, and the number of administrations of the bosentan extended-release oral preparation according to an exemplary embodiment of the present invention can be reduced, with the preparation preferably being administered once a day.

For administration once a day, the extended release preparation for oral administration of bosentan according to an exemplary embodiment of the present invention may contain the active ingredient and the sustained release excipient to be extended-released. That is, the active ingredient may be mixed with the sustained release excipient which are to be formulated into a single formulation, and thus the single formulation may be extended-released. At this time, the extended release preparation for oral administration of bosentan may be a tablet. The tablet may be a plain tablet or coated tablet.

In addition, the extended release preparation for oral administration of bosentanaccording to an exemplary embodiment of the present invention may include an extended-release section containing the active component and the sustained release excipient; and a non-extended-release section containing the same active ingredient as that contained in the extended-release section, and discriminated from the extended-release section.

The latter can have a continuous effect by continuously releasing the active ingredient contained in the extended-release section after the active ingredient of the non-extended-release section is released prior tothat the active ingredient of the extended-release section.

The active ingredient contained in the non-extended-release section may have a content of 0.1 to 50 parts by weight, and preferably 10 to 50 parts by weight, with respect to 100 parts by weight of the total active ingredients of the extended release preparation for oral administration of bosentan. In addition, the active ingredient contained in the extended-release section may be 50 to 99.9 parts by weight, and preferably 50 to 90 parts by weight with respect to 100 parts by weight of the total active ingredients of the extended release preparation for oral administration of bosentan. Within the above range, concerns about side effects due to a rapid increase in a blood concentration may be reduced, and concerns about a shorter duration of a drug effect may also be reduced.

The non-extended-release section may further include a disintegrant to increase a releaseg rate of the active ingredient as a result of fast disintegration.

The active ingredient contained in the extended-release section may be released from the intestine rather than the stomach in oral administration by further manufacturing an enteric coating layer including an enteric polymer on a surface of the extended-release section. The active ingredient of the extended-release section is delivered to the intestine with high pH without disintegration due to the enteric coating layer, while the active ingredient of the non-extended-release section is delivered to the intestine after disintegration. Since bosentan is more easily dissolved at high pH, the bosentan of the non-extended-release section which is delivered to the intestine and immediately released and disintegrated is dissolved and absorbed in the intestine at a high speed, and the active ingredient of the extended-release section delivered to the intestine without disintegration starts being released from the intestine, and is slowly released and absorbed over a long time. As a result, an initial blood concentration of the active ingredient is in an effective range, and maintained for a predetermined time, and thus an effect of the preparation to be administered once a day may be increased. The enteric polymer may be contained at 1 to 40 parts by weight, and preferably 3 to 30 parts by weight, with respect to 100 parts by weight of the extended release section. When the concentration is higher than the above range, the active ingredient is very slowly released and bioavailability may be reduced, and when the concentration is lower than the above range, the active ingredient is released from the stomach, thereby excessively increasing the initial blood concentration, resulting in side effects.

The extended release preparation for oral administration of bosentan may have a dissolution rate of the active ingredient of 10 to 50% after 1 hour, 20 to 70% after 2 hours, and 60% or more after 12 hours in a dissolution test performed by a paddle method using a phosphate buffer having pH 7.7 as an dissolution medium at a stirring speed of 50 rpm. As another condition, the extended release preparation for oral administration of bosentan may have a dissolution rate of the active ingredient of 10 to 50% after 1 hour, 50% or more after 10 hours, and 80% or more after 24 hours in a dissolution test by a paddle method using distilled water containing 1% lauryl sodium sulfate as an dissolution medium at a stirring speed of 50 rpm. After the concentration quickly reaches an effective blood concentration within the above range, the concentration is maintained to maintain a drug effect for a long time, and thus the number of administrations can be reduced to administration once a day. In addition, possible side effects can be reduced.

The extended release preparation for oral administration of bosentan may be prepared in various dosage forms including powders, granules, tablets, pellets, capsules, kits, etc., the extended-release section may be an extended-release powder, an extended-release granule, or an extended-release pellet, and the non- extended-release section may be a non-extended-release powder, a non-extended-release granule, or a non-extended-release pellet. A single formulation can be obtained by mixing the extended-release section with the non-extended-release section, or by mixing the sections, filling the mixture in a single capsule or manufacturing the mixture in the form of a tablet.

Although the present invention is not limited, the extended release preparation for oral administration of bosentan may be a tablet, and the tablet may be a monolayer tablet, a press-coated tablet or a multiple layer tablet.

The monolayer tablet may be a tablet prepared by mixing an extended-release granule constituting the extended release section with non- extended-release granule constituting a non-extended-release section, and manufacturing the mixture in the form of a tablet, resulting in one layer.

In the press-coated tablet, the extended release section becomes a core, and the non-extended-release section becomes a shell, and thus the blood concentration of the bosentan having a short half-life may be uniformly maintained for a long time, preferably 24 hours. The multiple layer tablet is composed of layers formed with the extended-release section and the non-extended-release section, respectively, and thus extended-release and non-extended-release may be easily controlled, and the blood concentration of the bosentan may be uniformly maintained for a long time, preferably 24 hours.

The active pharmaceutical ingredient(API) included in the extended release preparation for oral administration of bosentan may vary according to body weight, age, sex and the symptom of a patient, and in adults, bosentan as an active pharmaceutical ingredient may usually be administered at a dose of 32.25 mg, 62.5 mg, 125 mg, or 250 mg a day. Conventionally, the bosentan-containing preparation is administered twice a day, but the bosentan-containing preparation according to the present invention can be administered once a day, and thus ease of administration is increased. That is, according to a method of treating pulmonary hypertension according to an exemplary embodiment of the present invention including applying the extended release preparation for oral administration of bosentan according to the present invention to mammals including humans in need of administration thereof through oral administration once a day, pulmonary hypertension can be treated, and the ease of administration and patient compliance is improved. Moreover, the therapeutic effect is increased and the side effects are reduced. The mammals including humans in need of administration are subjects with pulmonary hypertension.

The extended release preparation for oral administration of bosentan may be formulated alone or in combination with pharmaceutically acceptable binders, diluents, disintegrants, surfactants, solubilizers, lubricants, dispersing agents, buffers, preservatives, flavoring agents, and coating agents as needed.

That is, the extended release preparation for oral administration of bosentan may be foumulated in a suitable form using additives such as pharmaceutically suitable and physiologically acceptable diluents, disintegrants, sweeteners, binders, coating agents, swelling agents, lubricants, glidants, or flavoring agents.

The disintegrant may be, for example, sodium starch glycolate, corn starch, potato starch, pregelatinized starch, bentonite, montmorillonite, veegum, microcrystalline cellulose, low-substituted hydroxypropyl cellulose, calcium carboxymethylcellulose, alginic acid, sodium croscarmellose, crospovidone, sodium bicarbonate, or citric acid, which may be used alone or in combination, but the present invention is not limited thereto.

To improve tablet ability of the granule and a dissolution rate of the tablet, a binder may be further included. The binder may be, but is not limited to, for example, microcrystalline cellulose, gelatine, polyethylene glycol, natural or synthetic gums, polyvinylpyrrolidone (povidone), polyvinyl alcohol, copovidone, progelatinized starch, starch, hydroxypropyl cellulose or hydroxypropyl methylcellulose.

A diluent may be further included. The diluent may be, but is not limited to, for example, starch, microcrystalline cellulose, lactose, glucose, mannitol, alkali earth metal salts, clay, polyethylene glycol or dicalcium phosphate.

A lubricant may be further included. The lubricant may be, but is not limited to, talc, stearic acid, magnesium stearate, calcium stearate, zinc stearate, sodium stearyl fumarate, sodium lauryl sulfate, hydrogenated vegetable oil, polyethylene glycol, glyceryl behenate, glyeryl monooleate, or glyceryl monostearate.

In addition, an emulsifier or surfactant such as lauryl sodium sulfate, a poloxamer, polysorbate, or a sorbitan derivative, or a solubilizer such as polyethylene glycol, a medium chain triglyceride, an isopropyl derivative, or a pyrrolidone derivative may be selectively included.

As an additional component, an antioxidant, a coloring agent, a flavoring agent, a preservative, or a sweetener may be used.

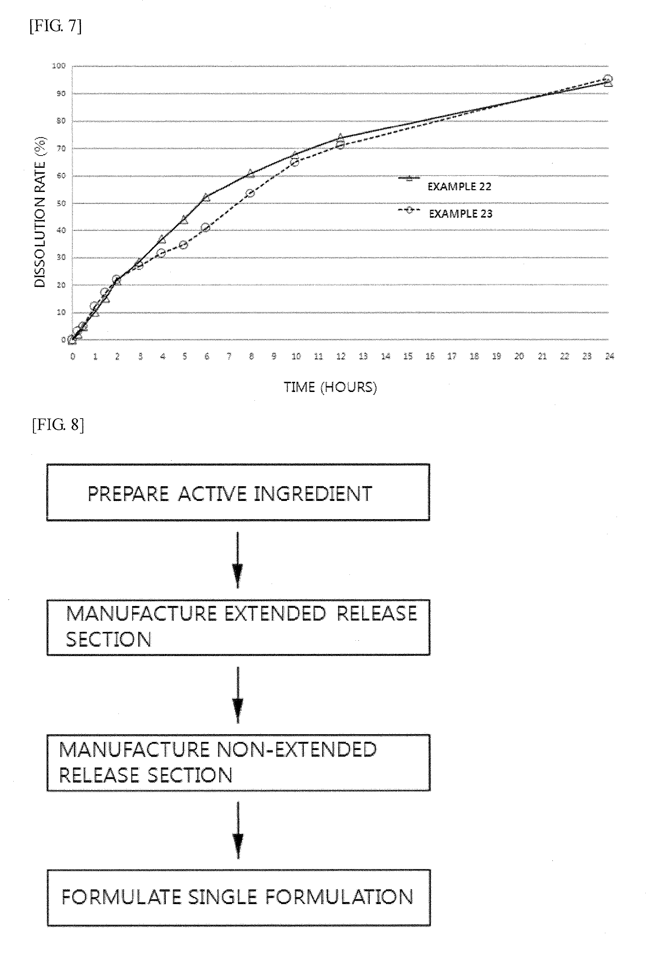

A method of manufacturing a bosentan extended-release oral preparation according to an exemplary embodiment of the present invention will be described in further detail with reference to FIG. 8. FIG. 8 is a flowchart illustrating a method of manufacturing an extended release preparation for oral administration of bosentan according to an exemplary embodiment of the present invention.

As shown in FIG. 8, the method of manufacturing an extended release preparation for oral administration of bosentan according to an exemplary embodiment of the present invention may be performed through preparation of an active ingredient, manufacture of an extended-release section, manufacture of a non-extended-release section and formulation into a single formulation. That is, the method may include preparing bosentan, a pharmaceutically acceptable salt thereof or a solvate thereof as an active ingredient; manufacturing an extended release section containing a sustained release excipient and a part of the active ingredient prepared in the first step; manufacturing a non-extended-release section containing the remaining content excluding the active ingredient contained in the extended-release section formed in the second step of the active ingredinet prepared in the first step as an active ingredient; and formulating the extended-release section manufactured in the second step and the non-extended-release section manufactured in the third step into a single formulation. The manufacture of the extended-release section and the manufacture of the non-extended-release section may be performed regardless of a sequence, simultaneously or in a different order than provided above.

To describe the preparation, for example, a press-coated tablet, in further detail, the extended release preparation for oral administration of bosentan may be manufactured by (A) preparing bosentan, a pharmaceutically acceptable salt thereof or a solvate thereof as an active ingredient; (B) manufacturing a extended-release granule containing a sustained release excipient and a part of the active ingredient prepared in step (A); (C) manufacturing a extended-release core by compressing the granule manufactured in step (B); (D) manufacturing a non-extended-release granule containing the remaining content excluding the active ingredient contained in the granule manufactured in step (B) of the active ingredient prepared in step (A); and (E) obtaining a press-coated tablet by compressing the extended-release core manufactured in step (C) encompassed by the non-extended-release granule manufactured in step (D).

The active ingredient in step (A) may be prepared by purchasing bosentan monohydrate. In step (B), a granule that can be manufactured in a tablet may be prepared by mixing a part of the bosentan monohydrate, a sustained release excipient and suitable additives. In step (C), a tablet may be prepared by pressing the extended-release granule manufactured in step (B) in a tablet, and then coated to control a releasing time of a tablet and improve stability. In step (D), a non-extended-release granule may be manufactured by adding and mixing an active ingredient and additives as needed (preferably an immediate release granule by adding at least one additive including a disintegrant). In step (E), a tablet may be manufactured by providing the non-extended-release granule prepared in step (D) to a press-coated tablet press. In this step, coating may be performed to improve a quality of the product. Steps (B), (C) and (D) may be performed regardless of an order, simultaneously, or in a different order than provided above.

A content of the active ingredient in step (B) may be 50 to 99.9 parts by weight, and the active ingredient in step (D) may be 0.1 to 50 parts by weight with respect to 100 parts by weight of the active ingredient prepared in step (A).

In addition, the extended-release core manufactured in step (C) may be manufactured by compressing the granule manufactured in step (B) and performing enteric coating. That is, the extended-release core may be manufactured by compressing the granule manufactured in step (B) or coating the granule with an aqueous polymer, and coating a surface with an enteric polymer to provide an enteric property.

In addition, the extended release preparation for oral administration of bosentan may be manufactured in a multiple layer tablet comprising the steps of (a) preparing bosentan, a pharmaceutically acceptable salt thereof or a solvate thereof as an active ingredient; (b) manufacturing a extended-release granule containing a sustained release excipient and a part of the active ingredient prepared in step (a); (c) manufacturing a non-extended-release granule containing the remaining content excluding the active ingredient contained in the granule manufactured in step (b) of the active ingredient prepared in step (a) as an active ingredient; and (d) obtaining a multiple layer tablet by compressing the extended-release granule manufactured in step (b) and the non-extended-release granule manufactured in step (c) in respective layers The above steps (a), (b) and (c) correspond the above steps (A), (B) and (D), respectively. In step (d), a multiple layer tablet may be prepared by compressing the granules manufactured in steps (b) and (c) respectively, using a multilayer tablet press to form respective layers. The multiple layer tablet may be manufactured as a bilayer tablet, or as a triple layer tablet including a placebo layer not containing an active ingredient. Steps (b) and (c) may be performed regardless of an order, simultaneously, or in a different order than provided above.

For example, the multiple layer tablet may include a placebo layer, and may be obtained by adding a granule for a placebo layer in step (d) and compressing the granule for a placebo layer with the extended-release granule and the non-extended-release granule to form respective layers.

In addition, another example of the extended release preparation for oral administration of bosentan may be manufactured by preparing bosentan, a pharmaceutically acceptable salt thereof or a solvate thereof as an active ingredient; preparing a sustained release excipient; and mixing the active ingredient and sustained release excipient to be formulated in a single formulation. The preparation of an active ingredient corresponds to the preparation according to an exemplary embodiment of the present invention, and the preparation of the sustained release excipient and the formulation in a single formulation corresponds to the manufacture of the extended release section and the formulation according to an exemplary embodiment. That is, the extended release preparation for oral administration of bosentan may be prepared as described in the above-described method according to an exemplary embodiment, excluding the manufacture of a non-extended-release section. The extended release preparation for oral administration of bosentan formulated by the above-described method may solely be extended-released.

In the formulation step, the granule may be directly prepared by mixing corresponding components or compressing the components with a roller, or by wet-granulation, melt-granulation, melt-congelation, or extrusion.

In addition, the preparation of the present invention may be prepared by a suitable method in the art, for example, by referencing Remington's Pharmaceutical Science (the latest issue), Mack Publishing Company, Easton, PA.

The descriptions of the extended release preparation for oral administration of bosentan and the descriptions of the method of manufacturing an extended release preparation for oral administration of bosentan are applied in the same manner unless there are contradiction.

Hereinafter, the present invention will be described in further detail with reference to Examples 1 to 28 and Experimental Examples 1 to 6. The compounds used in the following Examples and Experimental Examples were the highest quality commercially available compounds. Unless particularly described otherwise, materials listed in the Handbook of Pharmaceutical Excipient were purchased and used.

### <Example 1> Manufacture of an extended release preparation for oral administration of bosentan (press-coated tablet)

### 1) Manufacture of bosentan-containing extended release core

A preparation was manufactured in an amount of 1,000T with the composition and content of the extended-release layer described in Example 1 in Table 1. Bosentan monohydrate, carbomer, corn starch, and pregelatinized starch were sieved through a no. 20 sieve, and mixed for 10 minutes. Glyceryl behenate and magnesium stearate sieved through a no. 35 sieve were added to the mixture and mixed for 3 minutes, thereby manufacturing a bosentan-containing extended-release granule. The manufactured granule was pressed using a rotary press (ZPS-8, China) equipped with a 6.5 mm round punch. Separately, hydroxypropyl methylcellulose 2910 and polyethylene glycol 6,000 were dissolved in 80% ethanol, thereby preparing a primary coating solution. The extended-release core prepared as described above was put into a coating machine (SFC-30, Korea) and coated with a primary coating solution. Separately, an enteric coating layer was prepared by dissolving HPMC-AS in an ethanol-methylene chloride (1:1) mixed solution. The primary-coated tablet was coated with an enteric coating layer, and thus a bosentan-containing extended release core over which the enteric coating layer is formed was obtained.

### 2) Manufacture of bosentan-containing non-extended-release (immediate release) granule

A preparation was prepared in an amount of 1,000T with the composition and content of the extended-release layer described in Example 1 in Table 1. Bosentan monohydrate, corn starch, and pregelatinized starch were sieved through a no. 20 sieve, and mixed for 10 minutes. Separately, a binder solution was prepared by dissolving poylvinylpyrrolidone in water, and the mixture was kneaded with the binder solution. The kneaded product was dried at 50 °C to have a moisture content of less than 3.0%, and sieved through a no. 24 sieve. Sodium starch glycolate and glyceryl behenate were sieved through a no. 24 sieve, added to the sieved material and mixed for 10 minutes. Magnesium stearate sieved through a no. 35 sieve was added thereto, and mixed for 3 minutes, thereby manufacturing a bosentan-containing immediate release granule.

### 3) Manufacture of bosentan-containing extended- release tablet

The bosentan-containing extended-release core manufactured in 1) and the bosentan-containing immediate release granule manufactured in 2) were provided to a press-coated tablet press (RUD-1, Germany) equipped with a 12 mm round punch and pressed into a press-coated tablet. Separately, hydroxypropyl methylcellulose 2910, titanium oxide, polyethylene glycol 400, and iron oxide-yellow were dissolved in 80% ethanol, thereby preparing a coating solution. The pressed tablet was put into a coating machine (SFC-30F, Korea), and coated with a coating solution, thereby obtaining a bosentan-containing extended-release tablet.

### <Example 2> Manufacture of an extended release preparation for oral administration of bosentan (press-coated tablet)

An extended release preparation for oral administration of bosentan was manufactured as described in Example 1, except that hydroxypropyl methylcellulose 2208 was used instead of a carbomer in preparation of a bosentan-containing extended-release core with the composition and content of Example 2 in Table 1.

### <Example 3> Manufacture of an extended release preparation for oral administration of bosentan (press-coated tablet)

An extended release preparation for oral administration of bosentan was manufactured as described in Example 1, except that polyethylene oxide was used instead of a carbomer in preparation of a bosentan-containing extended-release core with the composition and content of Example 3 in Table 1.

### <Examples 4 and 5> Manufacture of an extended release preparation for oral administration of bosentan (press-coated tablet)

An extended release preparation for oral administration of bosentan was manufactured as described in Example 1, except that an amount of a carbomer was increased in preparation of a bosentan-containing extended-release core with the composition and content of Examples 4 and 5 in Table 1.

### <Examples 6 and 7> Manufacture of an extended release preparation for oral administration of bosentan (press-coated tablet)

An extended release preparation for oral administration of bosentan was manufactured as described in Example 1, except that a content of bosentan monohydrate in an extended-release core or an immediate release granule was changed to the composition and content of Examples 6 and 7 in Table 1.

**<Table 1>**

| Classification | Use | Name of component | Amount of use (mg per tablet) | | | | | | |
|---|---|---|---|---|---|---|---|---|---|
| | | | Example 1 | Example 2 | Example 3 | Exampie 4 | Example 5 | Exampie 6 | Example 7 |
| Extended-release layer | API | Bosentan monohydrate | 64.541 | 64.541 | 64.541 | 64.541 | 64.541 | 70.995 | 77.449 |
| | Sustained release excipient | Carbomer | 19.2 | - | - | 29.2 | 39.2 | 19.2 | 19.2 |
| | | Hydroxypropyl methylcellulose 2208 | - | 19.2 | - | - | - | - | |
| | | Polyethylene oxide | - | - | 19.2 | - | - | - | |
| | diluent | Corn starch | 10.259 | 10.259 | 10.259 | 10.259 | 10.259 | 10.259 | 10.259 |
| | diluent | Pregelatinized starch | 5.0 | 5.0 | 5.0 | 5.0 | 5.0 | 5.0 | 5.0 |
| | Lubricant | Glyceryl behenate | 4.0 | 4.0 | 4.0 | 4.0 | 4.0 | 4.0 | 4.0 |
| | Lubricant | Magnesium stearate | 2.0 | 2.0 | 2.0 | 2.0 | 2.0 | 2.0 | 2.0 |
| | Coating agent | Hydroxypropyl methylcellulose 2910 | 4.5 | 4.5 | 4.5 | 4.5 | 4.5 | 4.5 | 4.5 |
| | Plasticizer | Polyethylene glycol 6,000 | 0.5 | 0.5 | 0.5 | 0.5 | 0.5 | 0.5 | 0.5 |
| | Coating solvent | Distilled water (volatile) | (10.0) | (10.0) | (10.0) | (10.0) | (10.0) | (10.0) | (10.0) |
| | Coating solvent | Ethanol (volatile) | (40.0) | (40.0) | (40.0) | (40.0) | (40.0) | (40.0) | (40.0) |
| | Enteric coating agent | HPMC-AS | 10.0 | 10.0 | 10.0 | 10.0 | 10.0 | 10.0 | 10.0 |
| | Coating solvent | Ethanol (volatile) | (100.0) | (100.0) | (100.0) | (100.0) | (100.0) | (100.0) | (100.0) |
| | Coating solvent | Methylene chloride (volatile) | (100.0) | (100.0) | (100.0) | (100.0) | (100.0) | (100.0) | (100.0) |
| Immediate release layer | API | Bosentan monohydrate | 64.541 | 64.541 | 64.541 | 64.541 | 64.541 | 58.087 | 51.663 |
| | diluent | Corn starch | 225.459 | 225.459 | 225.459 | 225.459 | 225.459 | 225.459 | 225.459 |
| | diluent | Pregelatinized starch | 110.0 | 110.0 | 110.0 | 110.0 | 110.0 | 110.0 | 110.0 |
| | Binder | Polyvinylpyrrolidone | 12.0 | 12.0 | 12.0 | 12.0 | 12.0 | 12.0 | 12.0 |
| | Binding solvent | distilled water (volatile) | (50.0) | (50.0) | (50.0) | (50.0) | (50.0) | (50.0) | (50.0) |
| | Disintegrant | Sodium starch glyconate | 18.0 | 18.0 | 18.0 | 18.0 | 18.0 | 18.0 | 18.0 |
| | Lubricant | Glyceryl behenate | 12.0 | 12.0 | 12.0 | 12.0 | 12.0 | 12.0 | 12.0 |
| | Lubricant | Magnesium stearate | 8.0 | 8.0 | 8.0 | 8.0 | 8.0 | 8.0 | 8.0 |
| Final coating | Coating agent | Hydroxypropyl methylcellulose 2910 | 14.0 | 14.0 | 14.0 | 14.0 | 14.0 | 14.0 | 14.0 |
| | Plasticizer | Polyethylene glycol 400 | 2.0 | 2.0 | 2.0 | 2.0 | 2.0 | 2.0 | 2.0 |
| | Coloring agent | Iron oxide-yellow | 1.0 | 1.0 | 1.0 | 1.0 | 1.0 | 1.0 | 1.0 |
| | Coloring agent | Titanium oxide | 3.0 | 3.0 | 3.0 | 3.0 | 3.0 | 3.0 | 3.0 |
| | Coating solvent | Ethanol (volatile) | (160.0) | (160.0) | (160.0) | (160.0) | (160.0) | (160.0) | (160.0) |
| | Coating solvent | Distilled water (volatile) | (40.0) | (40.0) | (40.0) | (40.0) | (40.0) | (40.0) | (40.0) |
| Total (mg) | | | 590.0 | 590.0 | 590.0 | 600.0 | 610.0 | 590.0 | 590.0 |

### <Example 8> Manufacture of an extended release preparation for oral administration of bosentan (bilayer tablet)

### 1) Manufacture of bosentan-containing extended-release granule

A preparation was manufactured in an amount of 1,000T with the composition and content of the extended-release layer described in Example 8 in Table 2. Bosentan monohydrate, polyvinylpyrrolidone (66.7 wt% of total amount of use), colloidal silicon dioxide, pregelatinized starch, mannitol, and sodium lauryl sulfate were sieved through a no. 20 sieve, and mixed for 10 minutes. Separately, polyvinylpyrrolidone (33.3 wt% of total amount of use) was dissolved in distilled water, thereby preparing a binder solution, and added to and kneaded with the mixture. The kneaded granule was dried using a fluidized bed dryer (SFC-lab, Freund, Japan) to have a moisture content of less than 3%. The dried granule was sieved through a no. 20 sieve. Hydroxypropyl methylcellulose (viscosity: 4,000 mPa s) sieved through a no. 20 sieve was added to the sieved product and mixed for 20 minutes. After the mixing was completed, magnesium stearate and glyceryl behenate, which were sieved through a no. 35 sieve, were added and mixed for 10 minutes, and thus a bosentan-containing extended-release granule was manufactured.

### 2) Manufacture of bosentan-containing non-extended-release (immediate release) granule

A preparation was manufactured in an amount of 1,000T with the composition and content of the extended-release layer described in Example 8 in Table 2. Bosentan monohydrate, mannitol (81.1 wt% of total amount of use) and colloidal silicon dioxide (50.0 wt% of total amount of use) were sieved through a no. 20 sieve, and mixed for 10 minutes. Separately, polyvinylpyrrolidone was dissolved in distilled water, thereby preparing a binder solution, and added to and kneaded with the mixture. The kneaded granule was dried using a fluidized bed dryer (SFC-lab, Freund, Japan) to have a moisture content of less than 3%. The dried granule was sieved through a no. 20 sieve. Mannitol (50.0 wt% of total amount of use), crospovidone and colloidal silicon dioxide (50.0 wt% of total amount of use), which were sieved through a no. 20 sieve, were added to the sieved product and mixed for 5 minutes. After the mixing was completed, magnesium stearate sieved through a no. 35 sieve was added and mixed for 3 minutes, and thus a bosentan-containing non- extended-release granule was manufactured.

### 3) Manufacture of bosentan-containing extended-release tablet

The bosentan-containing extended-release granule manufactured in 1) and the bosentan-containing immediate release granule manufactured in 2) were provided to a multiple layer tablet press (MRC37T, Sejong, Korea) equipped with a major axis 15.3 mm rectangular punch to be pressed as a bilayer tablet. Separately, Kollicoat IR White II (BASF) was dissolved in distilled water, thereby preparing a coating solution. The prepared tablet was added to a coating machine (SFC-30F, Korea) and coated with a coating solution.

### <Example 9> Manufacture of extended release preparation for oral administration of bosentan (bilayer tablet)

An extended release preparation for oral administration of bosentan was manufactured as described in Example 8, except that hydroxypropyl methylcellulose (100 mPa s) was used instead of hydroxypropyl methylcellulose (viscosity: 4,000 mPa s) in preparation of a bosentan-containing extended-release granule with the composition and content of Example 9 in Table 2.

### <Example 10> Manufacture of an extended release preparation for oral administration of bosentan (bilayer tablet)

An extended release preparation for oral administration of bosentan was manufactured as described in Example 8, except that hydroxypropyl methylcellulose (15,000 mPa s) was used instead of hydroxypropyl methylcellulose (viscosity: 4,000 mPa s) in preparation of a bosentan-containing extended-release granule with the composition and content of Example 10 in Table 2.

### <Example 11> Manufacture of an extended release preparation for oral administration of bosentan (bilayer tablet)

An extended release preparation for oral administration of bosentan was manufactured as described in Example 8, except that hydroxypropyl methylcellulose (100,000 mPa s) was used instead of hydroxypropyl methylcellulose (viscosity: 4,000 mPa s) in preparation of a bosentan-containing extended-release granule with the composition and content of Example 11 in Table 2.

### <Example 12> Manufacture of an extended release preparation for oral administration of bosentan (bilayer tablet)

An extended release preparation for oral administration of bosentan was manufactured as described in Example 8, except that hydroxypropyl methylcellulose (200,000 mPa s) was used instead of hydroxypropyl methylcellulose (viscosity: 4,000 mPa s) in preparation of a bosentan-containing extended-release granule with the composition and content of Example 12 in Table 2.

### <Examples 13 and 14> Manufacture of an extended release preparation for oral administration of bosentan (bilayer tablet)

An extended release preparation for oral administration of bosentan was manufactured as described in Example 12, except that an amount of hydroxypropy lmethylcellulose (200,000 mPa · s) was changed in preparation of a bosentan-containing extended-release granule with the composition and content of Examples 13 and 14 in Table 2.

**[Table 2]**

| Classification | Use | Name of component | Amount of use (mg per tablet) | | | | | | |
|---|---|---|---|---|---|---|---|---|---|
| | | | Example 8 | Example 9 | Example 10 | Example 11 | Exampie 12 | Example 13 | Example 14 |
| Extended -release layer | API | Bosentan monohydrate | 103.266 | 103.266 | 103.266 | 103.266 | 103.266 | 103.266 | 103.266 |
| | Sustained release excipient | Hydroxypropyl methylcellulose (4,000 mPa s) | 150.0 | - | - | - | - | - | - |
| | | Hydroxypropyl methylcellulose (100 mPa s) | - | 150.0 | - | - | - | - | - |
| | | Hydroxypropyl methylcellulose (15,000 mPa s) | - | - | 150.0 | - | - | - | - |
| | | Hydroxypropyl methylcellulose (100,000 mPa s) | - | - | - | 150.0 | - | - | - |
| | | Hydroxypropyl methylcellulose (200,000 mPa s) | - | - | - | - | 150.0 | 120.0 | 180.0 |
| | Diluent | Mannitol | 249.734 | 249.734 | 249.734 | 249.734 | 249.734 | 249.734 | 249.734 |
| | Diluent | Pregelatinized starch | 20.0 | 20.0 | 20.0 | 20.0 | 20.0 | 20.0 | 20.0 |
| | Lubricant | Colloidal silicon dioxide | 12.0 | 12.0 | 12.0 | 12.0 | 12.0 | 12.0 | 12.0 |
| | Binder | Polyvinylpyrrolidone | 15.0 | 15.0 | 15.0 | 15.0 | 15.0 | 15.0 | 15.0 |
| | Binding solvent | Distilled water (volatile) | (60.0) | (60.0) | (60.0) | (60.0) | (60.0) | (60.0) | (60.0) |
| | Lubricant | Magnesium stearate | 8.0 | 8.0 | 8.0 | 8.0 | 8.0 | 8.0 | 8.0 |
| | Solubilizer | Sodium lauryl sulfate | 12.0 | 12.0 | 12.0 | 12.0 | 12.0 | 12.0 | 12.0 |
| | Lubricant | Glyceryl behenate | 30.0 | 30.0 | 30.0 | 30.0 | 30.0 | 30.0 | 30.0 |
| Immediate release layer | API | Bosentan monohydrate | 25.816 | 25.816 | 25.816 | 25.816 | 25.816 | 25.816 | 25.816 |
| | Diluent | Mannitol | 116.684 | 116.684 | 116.684 | 116.684 | 116.684 | 116.684 | 116.684 |
| | Binder | Polyvinylpyrrolidone | 4.0 | 4.0 | 4.0 | 4.0 | 4.0 | 4.0 | 4.0 |
| | Binding solvent | Distilled water (volatile) | (17.0) | (17.0) | (17.0) | (17.0) | (17.0) | (17.0) | (17.0) |
| | Lubricant | Colloidal silicon dioxide | 4.0 | 4.0 | 4.0 | 4.0 | 4.0 | 4.0 | 4.0 |
| | Disintegrant | Crospovidone | 7.0 | 7.0 | 7.0 | 7.0 | 7.0 | 7.0 | 7.0 |
| | Lubricant | Magnesium stearate | 2.5 | 2.5 | 2.5 | 2.5 | 2.5 | 2.5 | 2.5 |
| Final coating | Coating agent | Kollicoat IR White II | 20.0 | 20.0 | 20.0 | 20.0 | 20.0 | 20.0 | 20.0 |
| | Coating solvent | Distilled water (volatile) | (113.0) | (113.0) | (113.0) | (113.0) | (113.0) | (113.0) | (113.0) |
| Total (mg) | | | 780.0 | 780.0 | 780.0 | 780.0 | 780.0 | 780.0 | 780.0 |

### <Example 15> Manufacture of an extended release preparation for oral administration of bosentan (bilayer tablet)

An extended release preparation for oral administration of bosentan was manufactured as described in Example 12, except that hydroxyethyl cellulose was used instead of hydroxypropyl methylcellulose (200,000 mPa s) in preparation of a bosentan-containing extended-release granule with the composition and content of Example 15 in Table 3.

### <Example 16> Manufacture of an extended release preparation for oral administration of bosentan (bilayer tablet)

An extended release preparation for oral administration of bosentan was manufactured as described in Example 12, except that polyethylene oxide was further used in preparation of a bosentan-containing extended-release granule with the composition and content of Example 16 in Table 3.

### <Examples 17 to 19> Manufacture of an extended release preparation for oral administration of bosentan (bilayer tablet)

An extended release preparation for oral administration of bosentan was manufactured as described in Example 12, except that a ratio of contents of bosentan monohydrate included in an immediate release layer and a extended-release layer was changed to the composition and content of Examples 17, 18 and 19 in Table 3.

### <Example 20> Manufacture of an extended release preparation for oral administration of bosentan (bilayer tablet)

An extended release preparation for oral administration of bosentan was manufactured as described in Example 12, except that a content of bosentan monohydrate included in an immediate release layer was changed to the composition and content of Example 20 in Table 3.

### <Example 21> Manufacture of an extended release preparation for oral administration of bosentan (triple layer tablet)

A triple layer tablet including a placebo layer was manufactured with the composition and content of Example 21 in Table 3.

To form a granule for a placebo layer, mannitol and microcrystalline cellulose were sieved through a no. 20 sieve, and mixed for 20 minutes. After mixing, magnesium stearate sieved through a no. 35 sieve was added, and mixed for 3 minutes, thereby manufacturing the granule for a placebo layer. A triple layer tablet was obtained by providing a extended-release granule manufactured by the same method as that for the extended-release granule according to Example 12 as a first layer, a granule for a placebo layer as a second layer, and an immediate release granule manufactured by the same method as that for the immediate release granule according to Example 12 as a third layer. Afterward, a coating process was also performed by the same method as described in Example 12.

**[Table 3]**

| Classification | Use | Name of component | Amount of use (mg per tablet) | | | | | | |
|---|---|---|---|---|---|---|---|---|---|
| | | | Example 15 | Example 16 | Example 17 | Example 18 | Example 19 | Example 20 | Example 21 |
| extended release layer | API | Bosentan monohydrate | 103.266 | 103.266 | 116.174 | 90.357 | 77.450 | 206.532 | 103.266 |
| | Sustained release excipinet | Hydroxyethyl cellulose | 150.0 | - | - | - | - | - | - |
| | | Hydroxypropyl methylcellulose (200,000 mPa s) | - | 150.0 | 150.0 | 150.0 | 150.0 | 150.0 | 150.0 |
| | | Polyethylene oxide | - | 50.0 | - | - | - | - | - |
| | diluent | Mannitol | 249.734 | 249.734 | 236.826 | 262.643 | 275.550 | 146.468 | 249.734 |
| | diluent | Pregelatinized starch | 20.0 | 20.0 | 20.0 | 20.0 | 20.0 | 20.0 | 20.0 |
| | Lubricant | Colloidal silicon dioxide | 12.0 | 12.0 | 12.0 | 12.0 | 12.0 | 12.0 | 12.0 |
| | Binder | Polyvinylpyrrolidone | 15.0 | 15.0 | 15.0 | 15.0 | 15.0 | 15.0 | 15.0 |
| | Binding solvent | Distilled water (volatile) | (60.0) | (60.0) | (60.0) | (60.0) | (60.0) | (60.0) | (60.0) |
| | Lubricant | Magnesium stearate | 8.0 | 8.0 | 8.0 | 8.0 | 8.0 | 8.0 | 8.0 |
| | Solubilizer | Sodium lauryl sulfate | 12.0 | 12.0 | 12.0 | 12.0 | 12.0 | 12.0 | 12.0 |
| | Lubricant | Glyceryl behenate | 30.0 | 30.0 | 30.0 | 30.0 | 30.0 | 30.0 | 30.0 |
| Immediate release layer | API | Bosentan monohydrate | 25.816 | 25.816 | 12.908 | 38.725 | 51.632 | 51.632 | 25.816 |
| | Diluent | Mannitol | 116.684 | 116.684 | 129.592 | 103.775 | 90.868 | 90.868 | 116.684 |
| | Binder | Polyvinylpyrro lidone | 4.0 | 4.0 | 4.0 | 4.0 | 4.0 | 4.0 | 4.0 |
| | Binding solvent | Distilled water (volatile) | (17.0) | (17.0) | (17.0) | (17.0) | (17.0) | (17.0) | (17.0) |
| | Lubricant | Colloidal silicon dioxide | 4.0 | 4.0 | 4.0 | 4.0 | 4.0 | 4.0 | 4.0 |
| | Disintegrant | Crospovidone | 7.0 | 7.0 | 7.0 | 7.0 | 7.0 | 7.0 | 7.0 |
| | Lubricant | Magnesium stearate | 2.5 | 2.5 | 2.5 | 2.5 | 2.5 | 2.5 | 2.5 |
| Placebo layer | Diluent | Mannitol | - | - | - | - | - | - | 50.0 |
| | Diluent | Microcrystalline cellulose | - | - | - | - | - | - | 65.0 |
| | Lubricant | Magnesium stearate | - | - | - | - | - | - | 5.0 |
| Final coating | Coating agent | Kollicoat IR White II | 20.0 | 20.0 | 20.0 | 20.0 | 20.0 | 20.0 | 20.0 |
| | Coating solvent | Distilled water (volatile) | (113.0) | (113.0) | (113.0) | (113.0) | (113.0) | (113.0) | (113.0) |
| Total (mg) | | | 780.0 | 830.0 | 780.0 | 780.0 | 780.0 | 780.0 | 900.0 |

### <Example 22> Manufacture of an extended release preparation for oral administration of bosentan (monolayer tablet)

A preparation was manufactured in an amount of 1,000T with the composition and content of the extended-release layer described in Example 22 in Table 4. Bosentan monohydrate, polyvinylpyrrolidone (66.7 wt% of total amount), colloidal silicon dioxide, pregelatinized starch, mannitol, and sodium lauryl sulfate were sieved through a no. 20 sieve, and mixed for 10 minutes. Separately, a binder solution was prepared by dissolving polyvinylpyrrolidone (33.3 wt% of total amount), and applied to the mixture to knead. The kneaded granule was dried using a fluidized bed dryer (SFC-lab, Freund, Japan) to have a moisture content of less than 3%. The dried granule was sieved through a no. 20 sieve. Glyceryl behenate sieved through a no. 35 sieve was added to the sieved product, and mixed for 30 minutes. After mixing, magnesium stearate sieved through a no. 35 sieve was added, and mixed for 3 minutes, thereby manufacturing a extended-release granule. A tablet was obtained by pressing the granule after equipping a rotary tablet press (ZPS-8, China) with an 11.0 mm round punch. Separately, Kollicoat IR White II (BASF) was dissolved in distilled water, thereby obtaining a coating solution. The prepared tablet was provided to a coating machine (SFC-30F, Korea) and coated with the coating solution.

### <Example 23> Manufacture of an extended release preparation for oral administration of bosentan (monolayer tablet)

An extended release preparation for oral administration of bosentan was manufactured as described in Example 22, except that hydroxypropyl methylcellulose (200,000 mPa s) was used instead of glyceryl behenate with the composition and content of Example 23 in Table 4.

### <Example 24> Manufacture of anextended release preparation for oral administration of bosentan (monolayer tablet)

An extended release preparation for oral administration of bosentan was manufactured as described in Example 23, except that a different content of bosentan monohydrate was used with the composition and content of Example 24 in Table 4.

### <Examples 25 to 27> Manufacture of anextended release preparation for oral administration of bosentan (monolayer tablet)

An extended release preparation for oral administration of bosentan was manufactured as described in Example 23, except that a different viscosity of hydroxypropyl methylcellulose was used with the composition and content of Examples 25, 26 and 27 in Table 4.

### <Example 28> Manufacture of anextended release preparation for oral administration of bosentan (monolayer tablet)

An extended release preparation for oral administration of bosentan was manufactured as described in Example 22, except that ethylcellulose was used instead of glyceryl behenate with the composition and content of Example 28 in Table 4.

**[Table 4]**

| Classification | Use | Name of component | Amount of use (mg per tablet) | | | | | | |
|---|---|---|---|---|---|---|---|---|---|
| | | | Example 22 | Example 23 | Example 24 | Example 25 | Example 26 | Example 27 | Example 28 |
| Extended - release layer | API | Bosentan monohydrate | 129.082 | 129.082 | 258.164 | 129.082 | 129.082 | 129.082 | 129.082 |
| | Sustained release excipinet | Glyceryl behenate | 150.0 | - | - | - | - | - | - |
| | | Hydroxypropyl methylcellulose (200,000 mPa s) | - | 150.0 | 150.0 | - | - | - | - |
| | | Hydroxypropyl methylcellulose (100,000 mPa s) | - | - | - | 150.0 | - | - | - |
| | | Hydroxypropyl methylcellulose (15,000 mPa s) | - | - | - | - | 150.0 | - | - |
| | | Hydroxypropyl methylcellulose (4,000 mPa s) | - | - | - | - | - | 150.0 | - |
| | | Ethylcellulose | - | - | - | - | - | - | 150.0 |
| | Diluent | Mannitol | 203.918 | 203.918 | 124.836 | 203.918 | 203.918 | 203.918 | 203.918 |
| | Diluent | Progelatinated starch | 20.0 | 20.0 | 20.0 | 20.0 | 20.0 | 20.0 | 20.0 |
| | Lubricant | Colloidal silicon dioxide | 12.0 | 12.0 | 12.0 | 12.0 | 12.0 | 12.0 | 12.0 |
| | Binder | Polyvinylpyrro lidone | 15.0 | 15.0 | 15.0 | 15.0 | 15.0 | 15.0 | 15.0 |
| | Binding solvent | Distilled water (volatile) | (60.0) | (60.0) | (60.0) | (60.0) | (60.0) | (60.0) | (60.0) |
| | Lubricant | Magnesium stearate | 8.0 | 8.0 | 8.0 | 8.0 | 8.0 | 8.0 | 8.0 |
| | Solubilizer | Sodium lauryl sulfate | 12.0 | 12.0 | 12.0 | 12.0 | 12.0 | 12.0 | 12.0 |
| Final coating | Coating agent | Kollicoat IR White II | 20.0 | 20.0 | 20.0 | 20.0 | 20.0 | 20.0 | 20.0 |
| | Coating solvent | Distilled water (volatile) | (113.0) | (113.0) | (113.0) | (113.0) | (113.0) | (113.0) | (113.0) |
| Total (mg) | | | 570.0 | 570.0 | 620.0 | 570.0 | 570.0 | 570.0 | 570.0 |

### <Experimental Example 1> Extended release preparation for oral administration of bosentan assay and uniformity of contents test (1)

The extended release preparation for oral administration of bosentan (press-coated tablets) prepared in Examples 1 to 7 of the present invention were subjected to measurement of an assay in the preparation and an uniformity of contents value between the preparations by high performance liquid chromatography (HPLC) to be described below. The results are summarized in Table 5.

### <Conditions for HPLC analysis>

Detector: UV spectrophotometer (measurement wavelength: 272 nm)
Column: 4.6 mm×150 mm, 5 µm C8 column or a similar column
Column temperature: approximately 30 °C
Mobile phase: a solution of pH 4.2 buffer/acetonitrile mixture (43/57)
*pH 4.2 buffer: 6.73 g of anhydrous citric acid was dissolved in 1,000 mL of water, and adjusted to pH 4.2 with a sodium hydroxide solution
Flow rate: 1.0 mL/min
Injection volume: 10 µL
Run time: 40 min

**[Table 5]**

| Results of bosentan analysis (%; based on 95.0% ∼ 105.0%) | | | | | | |
|---|---|---|---|---|---|---|
| Example 1 | Example 2 | Example 3 | Example 4 | Example 5 | Example 6 | Example 7 |
| 99.8 | 100.2 | 99.5 | 101.1 | 100.8 | 99.7 | 99.8 |

| Values of bosentan uniformity of contents (based on 15% or less) | | | | | | |
|---|---|---|---|---|---|---|
| Example 1 | Example 2 | Example 3 | Example 4 | Example 5 | Example 6 | Example 7 |
| 2.7 | 3.5 | 3.1 | 3.4 | 3.2 | 2.9 | 3.0 |

As seen from Table 5, it was confirmed that uniform results of assay of bosentan were detected from the bosentan extended-release oral preparations (press-coated tablet) prepared according to the present invention even in a process of separately manufacturing an immediate release section and an extended-release section and mixing these sections. Particularly, according to an uniformity of contents, all press-coated tablets according to the present invention exhibited uniform contents of bosentan, and thus it was confirmed that deviation between patients could be minimized.

### <Experimental Example 2> Extended release preparation for oral administration of bosentan assay and uniformity of contents test (2)

The bosentan extended-release oral preparations (bilayer and triple layer tablets) prepared in Examples 8 to 21 of the present invention were subjected to measurement of an assay in the preparation and an uniformity of contents value between the preparations by the same method as described in Experimental Example 1. The results are summarized in Table 6.

**[Table 6]**

| Results of bosentan analysis (%; based on 95.0% ~ 105.0%) | | | | | | |
|---|---|---|---|---|---|---|
| Example 8 | Example 9 | Example 10 | Example 11 | Example 12 | Example 20 | Example 21 |
| 100.8 | 98.9 | 99.6 | 100.2 | 100.4 | 99.9 | 98.8 |

| Values of bosentan uniformity of contents test (based on 15% or less) | | | | | | |
|---|---|---|---|---|---|---|
| Example 8 | Example 9 | Example 10 | Example 11 | Example 12 | Example 20 | Example 21 |
| 3.6 | 3.8 | 4.2 | 3.9 | 4.5 | 2.7 | 4.9 |

As seen from Table 6, the bosentan extended-release oral preparations (bilayer and triple layer tablets) prepared in Examples 8 to 21 of the present invention showed similar results to those of Experimental Example 1. Particularly, a product having an uniformity of contents could be produced regardless of the kind of a sustained release excipient and a content ratio of the active ingredient. Accordingly, it was confirmed that all of the multiple layer tablets according to the present invention had uniform contents, and thus deviation between patients could be minimized.

### <Experimental Example 3> Extended release preparation for oral administration of bosentan assay and uniformity of contents test (3)

The bosentan extended-release oral preparations (monolayer tablets) prepared in Examples 22 to 28 of the present invention were subjected to measurement of an assay in the preparation and an uniformity of contents value between the preparations by the same method as described in Experimental Example 1. The results are summarized in Table 7.

**[Table 7]**

| Results of bosentan analysis (%; based on 95.0% ~ 105.0%) | | | | | | |
|---|---|---|---|---|---|---|
| Example 22 | Example 23 | Example 24 | Example 25 | Example 26 | Example 27 | Example 28 |
| 98.2 | 98.9 | 101.3 | 100.8 | 101.8 | 99.4 | 99.9 |

| Values of bosentan uniformity of contents test (based on 15% or less) | | | | | | |
|---|---|---|---|---|---|---|
| Example 22 | Example 23 | Example 24 | Example 25 | Example 26 | Example 27 | Example 28 |
| 4.8 | 4.1 | 3.3 | 3.8 | 4.4 | 4.1 | 4.7 |

As seen from Table 7, the bosentan extended-release oral preparations (monolayer tablets) prepared according to the present invention showed similar results to those of Experimental Examples 1 and 2. Particularly, even with different sustained release excipients, the contents of the oral preparations prepared by the preparation method of the present invention were uniform. Accordingly, it was confirmed that all of the monolayer tablets according to the present invention also had uniform contents, and thus deviation between patients could be minimized.

### <Experimental Example 4> Results of dissolution test (1)

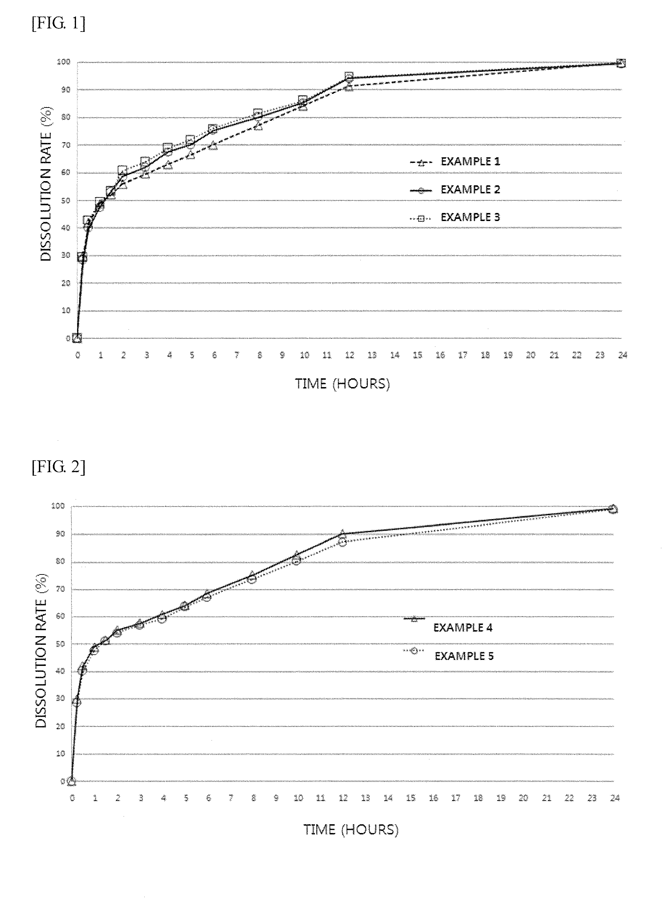

The extended release preparation for oral administration of bosentan (press-coated tablets) prepared in Examples 1 to 7 were subjected to a dissolution test under the following conditions. The results are shown in FIGS. 1 to 3.

### <Conditions for dissolution test>

Method of dissolution test: method of dissolution test according to the Korean pharmacopoeia (apparatus 2, paddle method)
Paddle speed: 50 rpm
Dissolution medium: 900 ml of pH 7.7 phosphate buffer
Sampling time: 0.25, 0.5, 1, 1.5, 2, 3, 4, 5, 6, 8, 10, 12, 24 hours

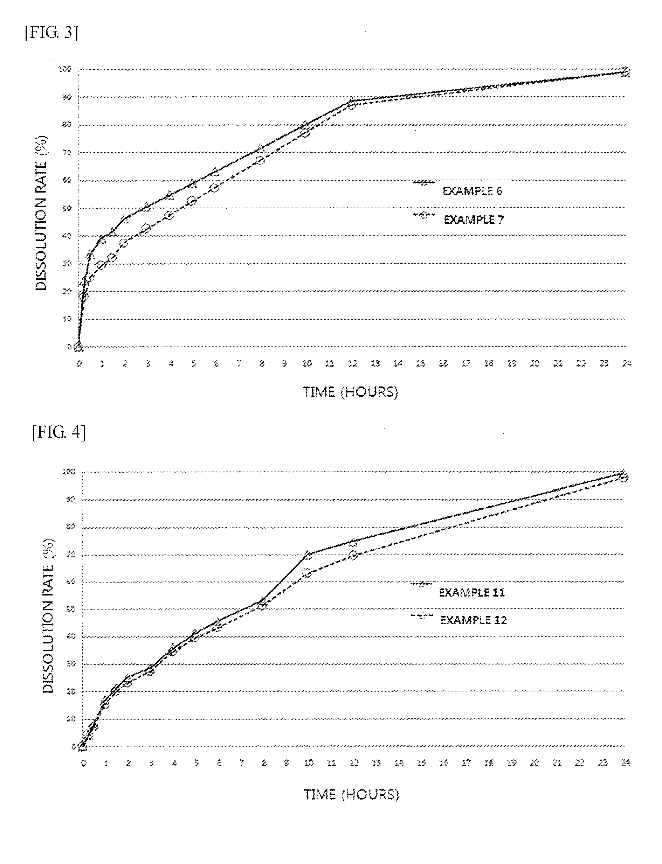

FIG. 1 is a graph of the results of a dissolution test for the extended release preparations for oral administration of bosentan according to Examples 1 to 3, FIG. 2 is a graph of the results of a dissolution test for the extended release preparations for oral administration of bosentan according to Examples 4 and 5, and FIG. 3 is a graph of the results of a dissolution test for the extended release preparations for oral administration of bosentan according to Examples 6 and 7. In each drawing, the x axis represents time, and the y axis represents a dissolution rate (%).

As seen from FIGS. 1, 2 and 3, in the examples of press-coated tablets according to the present invention, there were differences in total dissolution rates according to the kind and amount of the sustained release excipient, and the amounts of active ingredients included in the non-extended release (immediate release) section and the extended release section, but in the dissolution test according to the paddle method using a phosphate buffer (pH 7.7) as an dissolution medium, the active ingredient had a dissolution rate after one hour of 10∼50%, a dissolution rate after 2 hours of 20∼70%, and a dissolution rate after 12 hours of 60% or more. It was confirmed that the immediate release was rapidly dissolved until 1 hour with no influence from the extended-release section. In addition, the dissolution of the extended-release section exhibited after an hour showed a dissolution pattern with zero-order kinetics in which an amount of the dissolved active ingredient per unit time was uniform.

### <Experimental Example 5> Results of dissolution test (2)

The extended release preparations for oral administration of bosentan (press-coated tablets) prepared in Examples 8 to 21 were subjected to a dissolution test under the following conditions. The results are shown in FIGS. 4 to 6.

### <Conditions for dissolution test>

Method of dissolution test: method of dissolution test according the Korean pharmacopoeia (apparatus 2, paddle method)
Paddle speed: 50 rpm
Dissolution medium: 900 ml of pH 7.7 phosphate buffer
Sampling time: 0.25, 0.5, 1, 1.5, 2, 3, 4, 5, 6, 8, 10, 12, 24 hours

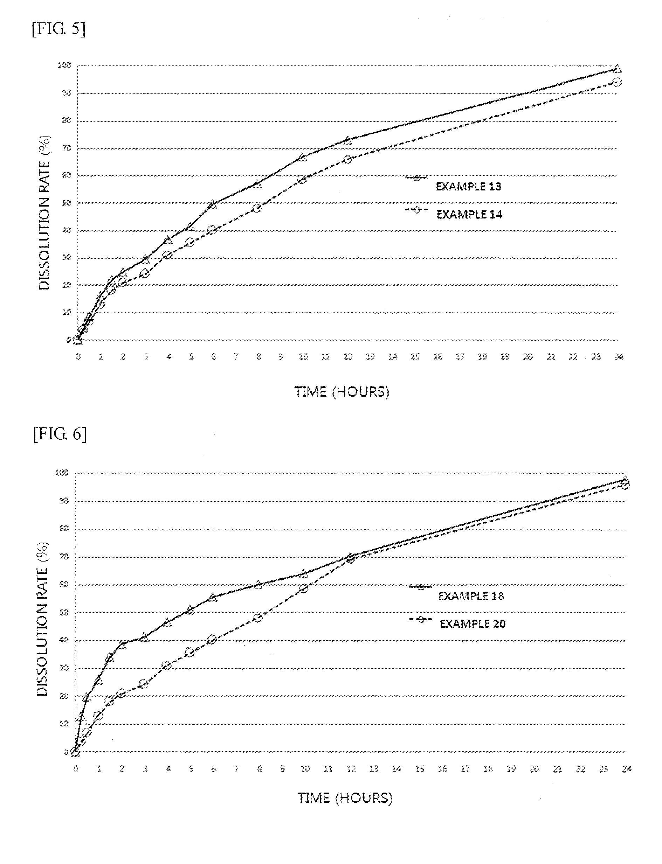

FIG. 4 is a graph of the results of a dissolution test for the extended release preparations for oral administration of bosentan according to Examples 11 and 12, FIG. 5 is a graph of the results of a dissolution test for the extended release preparations for oral administration of bosentan according to Examples 13 and 14, and FIG. 6 is a graph of the results of a dissolution test for the extended release preparations for oral administration of bosentan according to Examples 18 and 20. In each drawing, the x axis represents time, and the y axis represents a dissolution rate (%).

As seen from FIGS. 4, 5 and 6, in the examples of multiple layer tablets according to the present invention, there were differences in total dissolution rates according to the kind and amount of the sustained release excipient, the amounts of active ingredients included in the non-extended-release (immediate release) portion and the extended-release section and a total weight of the active ingredient, but in the dissolution test according to Experimental Example 5, the active ingredient had a dissolution rate after one hour of 10∼50%, a dissolution rate after 10 hours of 50% or more, and a dissolution rate after 12 hours of 80% or more. It was confirmed that the immediate release was rapidly dissolved until 1 hour with no influence from the extended release section. In addition, the dissolution of the extended-release section exhibited after an hour showed a dissolution pattern with zero-order kinetics in which an amount of the dissolved active ingredient per unit time was uniform.

### <Experimental Example 6> Results of dissolution test (3)

The extended release preparations for oral administration of bosentan (monolayer tablets) prepared in Examples 22 to 28 were subjected to a dissolution test under the same conditions as described in Experimental Example 5. The results are shown in FIG. 7.

FIG. 7 is a graph of a result of a dissolution test for the extended release preparations for oral administration of bosentan according to Examples 22 and 23. In each drawing, the x axis represents time, and the y axis represents a dissolution rate (%).

As seen from FIG. 7, in the examples of monolayer tablets according to the present invention, there were differences in total dissolution rates according to the kind and amount of the sustained release excipient, but the active ingredient had a dissolution rate after one hour of 10∼50%, a dissolution rate after 10 hours of 50% or more, and a dissolution rate after 12 hours of 80% or more. As a result, it was confirmed that a bosentan-containing extended-release preparation with zero-order kinetics could be prepared through the preparation of a monolayer tablet.

As seen from the above description, the preparation of the present invention can increase patient compliance through a decrease in administration cycle, and further reduce possibility of the occurrence of side effects caused by missing a suitable administration time due to extension of a blood half-life and a time to the maximum blood concentration of a drug through extended-release of the active ingredient.

That is, as a new extended release preparation for oral administration of bosentan exhibiting an excellent effect for 24 hours by administration once-a-day has been developed, the principal objects of the present invention such as ease of administration and compliance of a patient with pulmonary hypertension can be achieved and a new method for treating pulmonary hypertension using bosentan can be proposed.

As a result, a new extended release preparation for oral administration that can improve ease of administration by applying a sustained release excipient to bosentan solely developed to improve absorption through improvement of ease or solubility of the administration through improvement in dispersity according to the present invention, a method of manufacturing the same, and a treating method using the same can be provided.

### [Industrial Applicability]

According to the present invention, the number of administrations of bosentan, a pharmaceutically acceptable salt thereof or a solvate thereof can be reduced, and thus ease of administration can be improved, resulting in enhancing patient compliance.

## Claims

1. An extended release preparation for oral administration of bosentan, comprising:
bosentan, a pharmaceutically acceptable salt thereof or a solvate thereof as an active ingredient; and
a sustained release excipient.

2. The extended release preparation according to claim 1, wherein the active ingredient is bosentan monohydrate.

3. The extended release preparation according to claim 1, wherein the sustained release excipient is at least one selected from a hydrophilic polymer, a water-insoluble polymer and a lipid material.

4. The extended release preparation according to claim 3, wherein the hydrophilic polymer is at least one selected from hydroxyethyl cellulose, hydroxypropyl cellulose, hydroxypropyl methylcellulose, polyvinylpyrrolidone, polyvinyl alcohol, polyethylene glycol, polyethylene oxide, and a carbomer.

5. The extended release preparation according to claim 3, wherein the water-insoluble polymer is at least one selected from ethylcellulose, cellulose acetate, a poly(ethylacrylate, methylmethacrylate) copolymer, and a poly(ethylacrylate, methylmethacrylate, trimethylammonioethyl methacrylate) copolymer.

6. The extended release preparation according to claim 3, wherein the lipid material is at least one selected from glyceryl behenate, glyceryl palmitostearate, glyceryl oleate, glyceryl stearate, and cetyl alcohol.

7. The extended release preparation according to claim 1, wherein the sustained release excipient is contained at 0.5 to 200 parts by weight with respect to 100 parts by weight of the active ingredient.

8. The extended release preparation according to claim 1, wherein, in a dissolution test by a paddle method using a phosphate solution (pH 7.7) as an dissolution medium at a stirring speed of 50 rpm, the active ingredient has a dissolution rate after 1 hour of 10 to 50%, a dissolution rate after 2 hours of 20 to 70%, and a dissolution rate after 12 hours of 60% or more.

9. The extended release preparation according to claim 1, wherein, in a dissolution test by a paddle method using distilled water containing 1.0% sodium lauryl sulfate as an dissolution medium at a stirring speed of 50 rpm, the active ingredient has a dissolution rate after 1 hour of 10 to 50%, a dissolution rate after 10 hours of 70% or more, and a dissolution rate after 24 hours of 80% or more.

10. The extended release preparation according to any one of claims 1 to 9, which is a tablet.

11. The extended release preparation according to any one of claims 1 to 9, which is administered once a day.

12. The extended release preparation according to any one of claims 1 to 9, which comprises:
an extended-release section containing the active ingredient and the sustained release excipient; and
a non-extended release section containing the same active ingredient as that contained in the extended release section, and discriminated from the extended-release section.

13. The extended release preparation according to claim 12, wherein a content of the active ingredient contained in the non- extended-release section is 0.1 to 50 parts by weight, and a content of the active ingredient contained in the extended-release section is 50 to 99.9 parts by weight with respect to 100 parts by weight of total active ingredients of the bosentan extended-release oral preparation.

14. The extended release preparation according to claim 12, wherein the non-extended-release section is immediately released.

15. The extended release preparation according to claim 14, wherein the non-extended-release section further contains a disintegrant.

16. The extended release preparation according to claim 12, wherein an enteric coating layer including an enteric polymer is further formed on a surface of the extended-release section.

17. The extended release preparation according to claim 16, wherein the enteric polymer is at least one selected from hydroxypropyl methylcellulose acetate succinate, hydroxypropyl methylcellulose phthalate, cellulose acetate phthalate, a 1:1 copolymer of poly(methacrylic acid, methylmethacrylate), celluloseacetate phthalate, a 1:2 copolymer of poly(methacrylic acid, methylmethacrylate), and a 1:1 copolymer of poly(methacrylic acid, ethylmethacrylate).

18. The extended release preparation according to claim 16, wherein the enteric polymer is included at 1 to 40 parts by weight with respect to 100 parts by weight of the extended release section.

19. The extended release preparation according to claim 12, which is a tablet.

20. The extended release preparation according to claim 19, wherein the tablet is a press-coated tablet, in which the extended-release section becomes a core, and the non- extended-release section becomes a shell.

21. The extended release preparation according to claim 19, wherein the tablet is a multiple layer tablet, and the extended-release section and the non- extended-release section are formed in different layers.

22. The extended release preparation according to claim 21, wherein the multiple layer tablet includes a placebo layer containing an active ingredient, in which the placebo layer forms a separate layer from each of the layers of the extended-release section and the non- extended-release section.

23. The extended release preparation according to claim 19, wherein the tablet is a monolayer tablet.

24. A method of manufacturing an extended release preparation for oral administration of bosentan, comprising:
preparing bosentan, a pharmaceutically acceptable salt thereof or a solvate thereof as an active ingredient;
manufacturing an extended-release section containing a sustained release excipient and a part of the active ingredient prepared in the first step;
manufacturing a non-extended-release section containing the remaining content excluding the active ingredient contained in the extended-release section prepared in the manufacture of a extended-release section of the active ingredient prepared in the first step as an active ingredient; and
formulating the extended-release section prepared in the second step and non-extended-release section prepared in the third step into a single formulation.

25. A method of manufacturing an extended release preparation for oral administration of bosentan, comprising:
(A) preparing bosentan, a pharmaceutically acceptable salt thereof or a solvate thereof as an active ingredient;
(B) manufacturing an extended-release granule containing a sustained release excipient and a part of the active ingredient prepared in step (A);
(C) manufacturing an extended-release core by compressing the granule manufactured in step (B);
(D) manufacturing a non-extended-release granule containing the remaining content excluding the active ingredient contained in the granule manufactured in step (B) of the active ingredient prepared in step (A) as an active ingredient; and
(E) obtaining a press-coated tablet by compressing the extended-release core manufactured in step (C) emcompassed by the non-extended-release granule manufactured in step (D).

26. The method according to claim 25, wherein the active ingredient in step (B) is contained at 50 to 99.9 parts by weight, and the active ingredient in step (D) is contained at 0.1 to 50 parts by weight with respect to 100 parts by weight of the active ingredient prepared in step (A).

27. The method according to any one of claims 25 and 26, wherein the extended-release core manufactured in step (C) further undergoes enteric coating after compression of the granule manufactured in step (B).

28. A method of manufacturing an extended release preparation for oral administration of bosentan, comprising:
(a) preparing bosentan, a pharmaceutically acceptable salt thereof or a solvate thereof as an active ingredient;
(b) manufacturing a extended-release granule containing a sustained release excipient and a part of the active ingredient prepared in step (a);
(c) manufacturing a non- extended-release granule containing the remaining content excluding the active ingredient contained in the granule manufactured in step (b) of the active ingredient prepared in step (a) as an active ingredient; and
(d) obtaining a multiple layer tablet by compressing layers of the extended-release granule manufactured in step (b) and the non-extended-release granule manufactured in step (c).

29. The method according to claim 28, wherein the multiple layer tablet includes a placebo layer, which is formed by compressing a granule for a placebo layer included in step (d), together with the extended-release granule and the non-extended-release granule, with the granule of the placebo layer, the extended-release granule and the non-extended-release granule formed in separate layers.

30. A method of manufacturing an extended release preparation for oral administration of bosentan, comprising:
preparing bosentan, a pharmaceutically acceptable salt thereof or a solvate thereof as an active ingredient;
preparing a sustained release excipient; and
mixing the active ingredient prepared in the first step with the sustained release excipient prepared in the second step to be formulated in a single formulation.

31. A method of treating pulmonary hypertension, comprising:
administering the extended release preparation for oral administration of bosentan according to any one of claims 1 to 9 to mammals including humans in need of the administration once a day.
